# EUROPEAN PATENT SPECIFICATION

(11) **EP 4 506 021 B1**
(45) Date of publication and mention of the grant of the patent: **09.12.2026**
(21) Application number: 22933690.4
(22) Date of filing: 23.12.2022
(51) Int. Cl.: A61L 31/04, A61K 9/51, A61K 38/00, A61L 31/14, A61P 7/04, A61P 17/02, A61P 41/00

(54) **ANTI-ADHESIVE MATERIAL, AND METHOD FOR PRODUCING ANTI-ADHESIVE MATERIAL**
ANTIHAFTMATERIAL UND VERFAHREN ZUR HERSTELLUNG VON ANTIHAFTMATERIAL
MATÉRIAU ANTIADHÉSIF ET PROCÉDÉ DE PRODUCTION DE MATÉRIAU ANTIADHÉSIF

(30) Priority: 23.03.2022 JP 2022046287
(43) Date of publication of application: 12.02.2025
(73) Proprietor: National Institute for Materials Science, Tsukuba-shi, Ibaraki 305-0047 (JP)
(72) Inventor: TAGUCHI, Tetsushi, Tsukuba-shi, Ibaraki 305-0047 (JP)
(74) Representative: J A Kemp LLP
(86) International application number: PCT/JP2022/047729
(87) International publication number: WO 2023/181563

(56) References cited:
- EP-A1- 2 626 086
- WO-A1-2019/045081
- WO-A1-2020/050102
- WO-A1-2020/137903
- MIZUNO, YOSUKE ET AL.: "Tissue-sealing and anti-adhesion properties of an in situ hydrogel of hydrophobically-modified Alaska pollock-derived gelatin", INTERNATIONAL JOURNAL OF BIOLOGICAL MACROMOLECULES, vol. 163, 2020, pages 2365 - 2373, XP086300432, DOI: 10.1016/j.ijbiomac.2020.09.084
- NISHIGUCHI, AKIHIRO ET AL.: "Underwater-adhesive microparticle dressing composed of hydrophobically-modified Alaska pollock gelatin for gastrointestinal tract wound healing", ACTA BIOMATERIALIA, vol. 99, 2019, pages 387 - 396, XP085897096, DOI: 10.1016/j.actbio.2019.08.040
- NISHIGUCHI AKIHIRO, KURIHARA YUKARI, TAGUCHI TETSUSHI: "Hemostatic, Tissue-Adhesive Colloidal Wound Dressing Functionalized by UV Irradiation", ACS APPLIED BIO MATERIALS, AMERICAN CHEMICAL SOCIETY, US, vol. 3, no. 3, 16 March 2020 (2020-03-16), US , pages 1705 - 1711, XP093094854, ISSN: 2576-6422, DOI: 10.1021/acsabm.0c00015
- MIZUTA, RYO ET AL.: "Evaluation of an octyl group-modified Alaska pollock gelatin-based surgical sealant for prevention of postoperative adhesion", ACTA BIOMATERIALIA, vol. 121, 2021, pages 328 - 338, XP086472127, DOI: 10.1016/j.actbio.2020.12.025
- TAGUCHI, TETSUSHI: "Development of surgical adhesives with anti-adhesion function after curing", UEHARA MEMORIAL FOUNDATION RESEARCH REPORTS, UEHARA MEMORIAL LIFE SCIENCE FOUNDATION, JP, vol. 35, 30 November 2020 (2020-11-30), JP , pages 1 - 5, XP009549383, ISSN: 2433-3441
- ITO, SHIMA ET AL.: "Prevention of Postoperative Adhesion with a Colloidal Gel Based on Decyl Group Modified Alaska Pollock Gelatin Microparticles", ACTA BIOMATERIALIA, vol. 149, 11 June 2022 (2022-06-11), pages 139 - 149, XP087148834, DOI: 10.1016/j.actbio.2022.06.014

## Description

### TECHNICAL FIELD

The present invention relates to an anti-adhesion material and a method of manufacturing the anti-adhesion material.

### BACKGROUND ART

Postoperative adhesion, in which an organ adheres to the abdominal wall during the healing process or due to infection at a surgical area, occurs frequently after surgical procedures. The incidence rate is as high as 67% to 93%, of which 15% to 18% are estimated to require re-operation. Postoperative adhesion causes pain and intestinal obstruction, and makes re-operation difficult. Some studies report that postoperative adhesion significantly increases re-operation times by an average of 18 minutes. Therefore, new strategies for preventing postoperative adhesion are needed.

Postoperative adhesion may be caused by overabundant deposition of fibrin between the abdominal wall and the organs due to severe inflammation at a wound area and subsequent migration of inflammatory cells including fibroblast cells and macrophages migrate 3 to 5 days later. Therefore, one of the promising strategies to prevent postoperative adhesion is to introduce a physical barrier material which can prevent fibrin deposition and the migration of inflammatory cells from other wound areas. Reported anti-adhesion materials (physical barrier materials) are roughly classified into 3 types: (1) a solution type; (2) a film type; (3) a hydrogel type.

(1) Solution-type anti-adhesion materials (agents) such as hyaluronic acid solutions have been shown to prevent adhesion of organs to the peritoneum, and (2) film-type anti-adhesion materials are also widely used in clinical practice. For example, films made of carboxy methyl cellulose/sodium hyaluronate (HA/CMC) are often used to prevent postoperative adhesion. Alternatively, (3) hydrogel-type anti-adhesion materials are characterized by excellent injectability, quick gelation behavior in situ, and good followability with tissue geometries. Carboxymethyl dextrin, amino group/aldehyde-modified hyaluronic acid, and the like have been reported as the hydrogel-type anti-adhesion materials.

Meanwhile the present investors have previously reported tissue adhesive microparticles (gelatin derivative microparticles) based on a decyl group modified Alaska pollock gelatin (for example, Patent Document 1). These particles can be used as an anti-adhesion material because of their excellent adhesive strength to living tissues when applied to wound dressings and other materials.

Patent Document 2 discloses a composition comprising two types of gelatin in particulate form (freeze-dried): hydrophobically modified gelatin and unmodified gelatin in a ratio of 1:1; 1:2; 1:3; 1:5; 1:7; or 1:10.

### CITATION LIST

### Patent Document

Patent Document 1: WO2020/137903
Patent Document 2: EP 2 626 086 A1

### SUMMARY OF THE INVENTION

### Technical Problem

However, conventionally known anti-adhesion materials have various problems. For example, (1) the solution-type anti-adhesion materials have low stability on tissues, resulting in premature absorption. (2) The film-type anti-adhesion materials are inflexible and may stick to surgical gloves. Particularly in laparoscopic surgery, their introduction into the abdominal cavity has been reported to be difficult due to their fragility. Moreover, (3) the hydrogel-type anti-adhesion materials form hydrogel in situ by mixing two solutions together using a dual syringe. The preparation of hydrogel requires two solutions, a pre-gel solution and a cross-linking solution, as well as a dedicated syringe, making the preparation process complicated.

Accordingly, there is a need for a new anti-adhesion material having excellent adhesiveness on tissues, adhesion stability in aqueous environments, and simple operability.

Patent Document 1 discloses that gelatin derivative microparticles have excellent tissue adhesiveness, underwater stability, and the like. Nonetheless, further improvements were needed for application in anti-adhesion materials. In addition, anti-adhesion materials need to be easily mass produced in industrial settings.

The present invention is made to solve the above problems. That is, an object of the present invention is to provide an anti-adhesion material which is excellent in adhesiveness on tissues and adhesion stability in aqueous environments, and has simple operability, and, in addition, can be easily mass produced in industrial settings.

### Solution to Problem

After extensive studies to solve the above problems, the present inventors find that the following configurations can solve the above problems.
[1] An anti-adhesion material comprising a particle,
   the particle including a cross-linked product of a gelatin mixture of a first gelatin and a second gelatin having a hydrocarbon group introduced into the first gelatin,
   a hydrocarbon group introduction ratio of the gelatin mixture being 30 mol% to 50 mol%,
   the second gelatin having a structure represented by the formula (1) described below.
[2] The anti-adhesion material according to [1], wherein L is a single bond or - C(O)- in the formula (1).
[3] The anti-adhesion material according to [2], wherein L is a single bond in the formula (1).
[4] The anti-adhesion material according to any one of [1] to [3], wherein R¹ represents a linear alkyl group, and R² represents a hydrogen atom.
[5] The anti-adhesion material according to any one of [1] to [4], wherein the hydrocarbon group introduction ratio of the second gelatin is higher than the hydrocarbon group introduction ratio of the gelatin mixture.
[6] The anti-adhesion material according to any one of [1] to [5], wherein the hydrocarbon group introduction ratio of the second gelatin is 35 mol% to 80 mol%.
[7] The anti-adhesion material according to any one of [1] to [6], wherein the hydrocarbon group introduction ratio of the gelatin mixture is 35 mol% to 45 mol%.
[8] The anti-adhesion material according to any one of [1] to [7], wherein a percentage of the mass of the second gelatin to the total mass of the first gelatin and the second gelatin is 35 mass% to 90 mass%.
[9] The anti-adhesion material according to any one of [1] to [8], wherein the first gelatin is an alkali-treated gelatin.
[10] The anti-adhesion material according to any one of [1] to [9], wherein the first gelatin is a gelatin with a low endotoxin content.
[11] The anti-adhesion material according to any of [1] to [10], wherein the first gelatin is derived from cold-water fish.
[12] The anti-adhesion material according to any one of [1] to [11], which is applicable to an affected area of a living body using a spray system.
[13] A method of manufacturing the anti-adhesion material according to any one of [1] to [12], the method comprising:
   dissolving the gelatin mixture of the first gelatin and the second gelatin in a good solvent to prepare a gelatin mixture solution;
   adding a poor solvent to the gelatin mixture solution to precipitate a first intermediate particle containing the gelatin mixture in the gelatin mixture solution;
   lyophilizing the gelatin mixture solution including the first intermediate particle to obtain a second intermediate particle; and
   cross-linking the gelatin mixture of the second intermediate particle to obtain a particle having the gelatin mixture cross-linked.
[14] The method of manufacturing the anti-adhesion material according to [13], comprising: heating the second intermediate particle to cross-link the gelatin mixture.

### Advantageous Effects of the Invention

The anti-adhesion material according to the present invention is excellent in adhesiveness on tissue and adhesion stability in aqueous environments, and has simple operability, and, in addition, can be easily mass produced in industrial settings.

### BRIEF DESCRIPTION OF THE DRAWINGS

FIG. 1 shows a schematic diagram illustrating a configuration of an anti-adhesion material (particles) according to the present embodiment and a method of manufacturing it.
FIG. 2 shows a schematic diagram illustrating a function of the present anti-adhesion material (particles) according to the present embodiment.
FIG. 3 shows a schematic diagram illustrating a function of the anti-adhesion material (particles) according to the present embodiment in a living body (rat).
FIG. 4 shows a schematic diagram illustrating an application of the anti-adhesion material (particles) according to the present embodiment using a spray system.
FIG. 5A shows SEM (Scanning Electron Microscope) images of particles produced in Example. The scale bar represents 10 µm.
FIG. 5B shows particle diameter distributions of particles produced in Example.
FIG. 6 shows photographs of the particles produced in Example being sprayed with an endoscopic spray device.
FIG. 7A shows graphs indicating the time dependency of water content (the relationship between hydration time and water content) of colloidal gels of the particles produced in Example.
FIG. 7B shows bright-field micrographs indicating aggregation behaviors of the particles produced in Example. The scale bar represents 20 µm.
FIG. 8A shows a schematic diagram illustrating the procedures for the *in vitro* tissue adhesion/non-adhesion tests in Example.
FIG. 8B shows graphs indicating the relationship between hydration time and adhesive strength in the *in vitro* tissue adhesion/non-adhesion tests in Example.
FIG. 8C shows images of hematoxylin-eosin (HE) stained tissues and colloidal gel fixed between the upper and lower jigs after the *in vitro* tissue adhesion and nonadhesion tests in Example.
FIG. 9A shows images of hematoxylin-eosin (HE) stained tissues and colloidal gels after the underwater stability tests in Example.
FIG. 9B shows graphs illustrating the remaining areas of colloidal gels on tissues after the underwater stability tests in Example.
FIG. 10A shows photographs illustrating the application experiments of the anti-adhesion material (particles) to a cecum-abdominal wall adhesion model of SD rats in Example. (a) A photograph of normal cecum (Cecum) and peritoneum (Peritoneal), (b) a photograph showing cecal abrasions and abdominal wall defects (labeled "Defect" in the figure) were formed, (c) a photograph of particles (MPs) produced in Example applied to the entire defective areas, (d) a photograph of colloidal gels formed on the defective areas by hydrating the particles produced in Example with physiological saline.
FIG. 10B shows photographs illustrating results from the application experiments of the anti-adhesion material (particles) to the cecum-abdominal wall adhesion model of SD rats in Example.
FIG. 10C shows graphs indicating the results (adhesion scores one week after application (W1, 1 week)) from the application experiments of the anti-adhesion material (particles) to the cecum-abdominal wall adhesion model of SD rats in Example.
FIG. 10D shows graphs indicating the results (adhesion scores two week after application (W2, 2 weeks)) from the application experiments of the anti-adhesion material (particles) to the cecum-abdominal wall adhesion model of SD rats in Example.

### DETAILED DESCRIPTION OF THE INVENTION

Below, the present invention will be described in detail.

Although the descriptions of the requirements of the components described below may be based on the representative embodiments of the present invention, the present invention shall not be limited to these embodiments.

It is noted that the ranges of numerical values expressed using "to" as used herein include the numerical values listed before and after "to" as the lower and upper limits.

When groups (groups of atoms) are described herein, those not explicitly indicated as substituted or unsubstituted encompass both those without substituents and those with substituents to the extent that an effect of the invention is achieved. For example, the term "alkyl group" encompasses not only alkyl groups without substituents (unsubstituted alkyl groups) but also alkyl groups with substituents (substituted alkyl groups). This also apples to other compounds as well.

The anti-adhesion material according to the present invention comprises a particle, the particle including a cross-linked gelatin mixture. The gelatin mixture includes a raw material gelatin (an example of a "first gelatin") and a hydrophobized gelatin (an example of a "second gelatin") having a hydrocarbon group introduced into the raw material gelatin.

### <Raw material gelatin (first gelatin)>

The raw material gelatin may be naturally-occurring or synthesized (including fermented, genetically modified, and the like). Alternatively, the raw material gelatin may be a naturally-occurring or synthesized gelatin subjected to some kind of modification. Specific examples include naturally-occurring gelatins obtainable from skin, bone, and tendon, and the like of mammal, bird, fish, and the like; modified gelatins prepared by treating naturally-occurring gelatins with acid or alkali (heat extracted if necessary); and the like. Among these, an alkali-treated gelatin is preferred because a particle showing a superior effect of the invention can be obtained.

As the raw material gelatin, a gelatin with a low endotoxin content is preferred. There is no particular limitation for the low-endotoxinized gelatins, and any known gelatin with a low endotoxin content can be used. Examples include those described in Japanese Patent Application Laid Open No. 2007-231225.

Gelatins derived from mammal include those derived from porcine and bovine. There is no particular limitation for gelatins derived from fish, but among these, gelatins derived from cold-water fish (cold-water fishes) such as salmon, trout, cod, Alaska pollock, sea bream, tilapia, and tuna, and the like (hereinafter referred to as "gelatins derived from cold-water fish") are preferred.

Gelatins derived from cold-water fish are macromolecules having two or more amino acids connected in a linear fashion. Gelatins derived from cold-water fish have 190 or less imino acid per 1000 constituent amino acids. More specifically, they have 80 or less hydroxyprolines and 110 or less prolines. It is thought that the room temperature fluidity of gelatins derived from cold-water fish may be attributed to the number of hydroxyprolines being 80 or less, or the number of prolines being 110 or less. It is thought that when either of the conditions is met, they undergo denaturation at around room temperature or lower, leading to flowability at room temperature.

A sea-bream gelatin has 73 hydroxyprolines and 108 prolines, and has a denaturation temperature of 302.5 K. A tilapia gelatin has 82 hydroxyprolines and 110 prolines, and has a denaturation temperature of 309 K. In contrast, a porcine gelatin has 95 hydroxyprolines and 121 prolines, and has a denaturation temperature of 316 K.

It is noted that gelatins derived from cold-water fish have amino acid sequences similar to those of animal gelatins, and may be easily decomposed by enzyme. They also have high biocompatibility.

There is no particular limitation for the molecular weight of the raw material gelatin, but the weight average molecular weight (Mw) thereof is preferably 5,000 to 100,000, more preferably 10,000 to 50,000, and even more preferably 20,000 to 40,000. It is noted that the term "weight average molecular weight" as used herein means a weight average molecular weight determined by gel permeation chromatography (GPC).

The raw material gelatin may consist of only one type of gelatin, or may be a mixture of two or more types of gelatins.

### <Hydrophobized gelatin (second gelatin)>

The term "hydrophobized gelatin" as used herein means a gelatin derivative having a hydrocarbon group introduced into the raw material gelatin. The hydrophobized gelatin has a structure represented by the following formula (1):

GltnNH-L-CHR¹R² (1),

wherein Gltn represents a residue of a gelatin, and L represents a single bond or a divalent linking group, and R¹ represents an alkyl group having 1 to 20 carbon atoms, and R² represents a hydrogen atom or an alkyl group having 1 to 20 carbon atoms.

In the formula (1), GltnNH- represents a structure derived from the raw material gelatin as described above. Therefore, the raw material gelatin is represented by GltnNH₂. -CHR¹R² represents a hydrocarbon group introduced into the raw material gelatin, and is introduced into the raw material gelatin through L (a single bond or a divalent linking group).

There is no particular limitation for the divalent linking group for L, but examples include -C(O)-; -C(O)O-; - OC(O)-; -O-; -S-; -N(R)- wherein R represents a hydrogen atom or a monovalent organic group, preferably a hydrocarbon group having 1 to 20 carbon atoms; an alkylene group, preferably an alkylene group having 2 to 10 carbon atoms; an alkenylene group, preferably an alkenylene group having 2 to 10 carbon atoms; combinations thereof; and the like. Among them, -C(O)- is preferred. L is preferably a single bond or -C(O)-.

In the formula (1), -CHR¹R² (a hydrocarbon group) is preferably bound to an ε-amino group of the raw material gelatin, more preferably bound to ε-amino group of lysine (Lys) in the raw material gelatin. Examples of a method of attaching *-CHR¹R² to an amino group, preferably the amino group of lysine with or without a linking group (directly in other words) include, for example, a so-called reductive amination reaction (a method using aldehyde or ketone), the Schotten-Baumann reaction (a method using acid chloride), and the like.

It is noted that the -NH- structure (a secondary animo group) in the formula (1) can be detected, for example, by the presence of a band near 3300 cm⁻¹ in an FT-IR (Fourier transform infrared absorption) spectrum.

There is no particular limitation for the hydrocarbon groups having 1 to 20 carbon atoms for R¹ and R² in the formula (1), but they include, for example, a chain hydrocarbon group having 1 to 20 carbon atoms, an alicyclic hydrocarbon group having 3 to 20 carbon atoms, an aromatic hydrocarbon group having 6 to 14 carbon atoms, and groups of combinations thereof.

When R² is 1 to 20 hydrocarbon groups, R² may or may not be identical to R¹. An alkyl group in R¹ and R² may be a linear chain, or may be a branched chain.

There is no particular limitation for the chain hydrocarbon group having 1 to 20 carbon atoms, but examples include a methyl group, an ethyl group, a propyl group, a butyl group, a hexyl group, an octyl group (or a capryl group), a nonyl group (or a pelargonyl group), a decyl group, a dodecyl group (or a lauryl group), a tetradecyl group (or a myristyl group), and the like. Among these, R¹ is preferably an alkyl group having 1 to 13 carbon atoms, more preferably an alkyl group having 7 to 12 carbon atoms, even more preferably an alkyl group having 8 to 11 carbon atoms, and in particular preferably an alkyl group having 9 to 11 carbon atoms because a particle having superior adhesiveness can easily be obtained. There is no particular limitation for R², but it is preferably a hydrogen atom.

Examples of the alicyclic hydrocarbon group having 3 to 20 carbon atoms include, for example, a cyclopropyl group, a cyclopentyl group, a cyclohexyl group, an adamantyl group, a norbornyl group, and the like.

There is no particular limitation for the aromatic hydrocarbon group having 6 to 14 carbon atoms, but examples include a phenyl group, a tolyl group, a naphthyl group, and the like.

There is no particular limitation for the combinations of the above groups, but they include, for example, an aralkyl group having 6 to 12 carbon atoms such as a benzyl group, a phenethyl group, a naphthylmethyl group, and a naphthylethyl group, and the like.

The total of carbon atoms in the hydrocarbon group (-CHR¹R²) is preferably 9 to 20, 9 to 18, or 9 to 14. In the hydrocarbon group (-CHR¹R²), it is preferred that R¹ be a linear alkyl group, and R² be a hydrogen atom or a linear alkyl group, and it is more preferred that R¹ be a linear alkyl group and R² be a hydrogen atom.

The hydrophobized gelatin represented by the formula (1) is preferably at least one selected from the group consisting of the following formulas (2) and (3), and is more preferably a gelatin derivative represented by the formula (2).

GltnNH-CHR¹R² (2)

GltnNH-C(O)- CHR¹R² (3)

The formula (2) represents a case where L is a single bond in the formula (1). In the formula (2), the hydrocarbon group (-CHR¹R²) is introduced into a gelatin residue Gltn of a raw material gelatin via an imino bond (-NH-).

The formula (3) represents a case where L is -C(O)- in the formula (1). In the formula (3), the hydrocarbon group (-CHR¹R²) is introduced into a gelatin residue Gltn of a raw material gelatin via an amido bond (-NHCO-).

In the formulas (2) and (3), the meanings of symbols as well as suitable forms are the same as in the formula (1) already described.

The hydrophobized gelatin may consist of only one type of gelatin derivative, or may be a mixture of two or more types of gelatin derivatives.

Here, the molar ratio of the content of imino groups to which alkyl groups are bound (*-NH-CHR¹R²) in the hydrophobized gelatin to the content of amino groups (-NH2) in the gelatin before hydrophobization (the raw material gelatin) is defined as a "hydrocarbon group introduction ratio."

There is no particular limitation for the hydrocarbon group introduction ratio of the hydrophobized gelatin as long as it has a value higher than that of the gelatin mixture described below. The hydrocarbon group introduction ratio of the hydrophobized gelatin may be 35 mol% to 80 mol%, 40 mol% to 70 mol%, or 45 mol% to 60 mol%. In other words, the ratio (molar ratio) of imino group/amino group in the hydrophobized gelatin may be 35/65 to 80/20, 40/60 to 70/30, or 45/55 to 60/40. If the hydrocarbon group introduction ratio of the hydrophobized gelatin is within the above range, the hydrocarbon group introduction ratio of the gelatin mixture described below can be easily adjusted to a specific range. As a result, appropriate hydrophobicity can be conferred on the anti-adhesion material (particles) according to the present embodiment.

It is noted that in an embodiment, the hydrocarbon group introduction ratio may be computed by the following formula by substituting values obtained by quantifying the number of amino groups in the raw material gelatin and the number of amino groups in the hydrophobized gelatin using the 2,4,6-trinitrobenzenesulfonic acid method (the TNBS method). 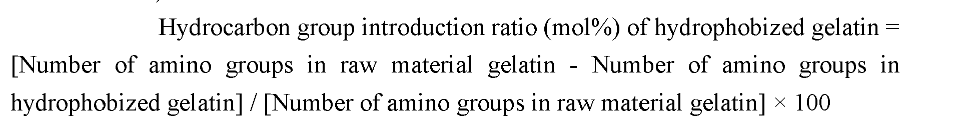 Hydrocarbon group introduction ratio (mol%) of hydrophobized gelatin = [Number of amino groups in raw material gelatin - Number of amino groups in hydrophobized gelatin] / [Number of amino groups in raw material gelatin] × 100

There is no particular limitation for the number hydrocarbon groups (-CHR¹R²) introduced into one molecule of the hydrophobized gelatin. Based on the molecular weight of the raw material gelatin, the number of hydrocarbon groups may be suitably adjusted so that the hydrocarbon group introduction ratio has a predetermined value. For example, the number of hydrocarbon groups in one molecule of the hydrophobized gelatin may be 5 to 10, 6 to 9, or 6 to 8.

There is no particular limitation for the molecular weight of the hydrophobized gelatin. The molecular weight of the hydrophobized gelatin is determined by the molecular weight of the raw material gelatin and the type and amount (number) of hydrocarbon groups introduced. Therefore, the range of a possible weight average molecular weight (Mw) of the hydrophobized gelatin is almost the same as the range of a possible weight average molecular weight of the raw material gelatin described above.

The hydrophobized gelatin as described above may be commercially available, or may be synthesized in house. A hydrophobized gelatin may be synthesized by introducing hydrophobic groups (hydrocarbon groups) into a raw material gelatin, for example, by the synthesis method disclosed in Patent Document 1.

### <Particles>

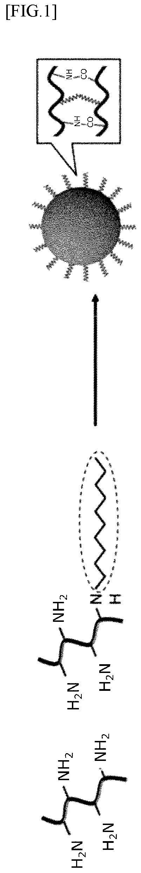

A particle according to the present embodiment comprises a cross-linked product of a gelatin mixture. The gelatin mixture is a mixture of the raw material gelatin (the first gelatin) and the hydrophobized gelatin (the second gelatin) described above (see FIG. 1). As described above, the raw material gelatin and the hydrophobized gelatin share the identical basic backbone. This may facilitate uniform proceeding of a cross-linking reaction in the mixture and a composition of the resulting particle (a cross-linked product) becomes uniform, making it easy to achieve an effect of the present embodiment as described below.

The hydrocarbon group introduction ratio of a gelatin mixture can be computed by the following Equation (I):
$Xm = Xc \left(A/100\right)$ (I)
Xm: the hydrocarbon group introduction ratio of a gelatin mixture (mol%);
Xc: the hydrocarbon group introduction ratio of a hydrophobized gelatin (mol%); and
A: the percentage of the mass of the second gelatin to the total mass of the raw material gelatin and the hydrophobized gelatin (mass%).

The hydrocarbon group introduction ratio of a gelatin mixture may be 30 mol% to 50 mol%, or may be 35 mol% to 45 mol%. If the hydrocarbon group introduction ratio of a gelatin mixture is in this range, appropriate hydrophobicity can be conferred on the anti-adhesion material (particles), and an effect of the present embodiment described below can be easily achieved.

There is no particular limitation for a mixing ratio of the raw material gelatin and the hydrophobized gelatin. The mixing ratio may be adjusted based on the hydrocarbon group introduction ratio of a hydrophobized gelatin. In one aspect, the hydrocarbon group introduction ratio of a gelatin mixture can be appropriately adjusted to have a specific value. For example, the percentage of the mass of the second gelatin (Xc) to the total mass of the raw material gelatin and the hydrophobized gelatin can be 35 mass% to 90 mass%, 35 mass% to 70 mass%, or 35 mass% to 50 mass%.

The particle according to the present embodiment comprises a cross-linked gelatin mixture (a cross-linked product of a gelatin mixture). As used herein, the term "cross-linked" does not include reversible physical cross-linked structures, but means cross-linked structures obtained by irreversible cross-linking reactions. Therefore, the term "cross-linked gelatin mixture (cross-linked product of a gelatin mixture)" has an irreversible cross-linked structure obtained by a cross-linking reaction. The cross-linking reaction may be induced by conferring energy on a gelatin mixture via heat, light, energy rays, and the like. The cross-linking reaction may also be induced by a cross linking agent. The cross-linking reaction for a cross-linked gelatin mixture can be induced by one or both of the above.

The particle may be produced by cross-linking a gelatin mixture with a cross-linking agent (a compound different from the raw material gelatin and the hydrophobized gelatin). It is preferred that no cross-linking agent be used. This is safe because no impurities are derived from the cross-linking agent. In this case, cross-linking is induced, for example, via a reaction between functional groups (-NH2, -OH, -SH, -COOH, and the like) in gelatin side chains. A particle containing a cross-linked gelatin mixture shows enhanced adhesive strength, and are thus more suitable for an anti-adhesion material and the like.

In an embodiment of the present invention, the particle may consist only of a cross-linked product of a gelatin mixture. Other components may also be included to the extent that an effect of the present invention can be obtained.

There is no particular limitation for the percentage (content) of the cross-linked product of a gelatin mixture. The percentage of the cross-linked product may be 100 mass%, 98 mass% or more, or 90 mass% or more, as it is easier to obtain a particle having a superior effect of the present invention.

There is no particular limitation for the other components which may be included in the particle, but they include, for example, solvents, buffering agents, colorants, preservatives, excipients, agents (antithrombotic agents, antibacterial agents, and growth factors, and the like), and the like.

There is no particular limitation for the average particle diameter of the particles according to the present embodiment, but it is, for example, 0.5 µm to 50 µm, preferably 1 µm to 30 µm, more preferably 1 µm to 10 µm. The term "average particle diameter" as used herein refers to a value obtained by measuring and averaging the particle diameters (longest diameters) of 100 randomly selected particles under an electron microscope.

### <Anti-adhesion material>

The particles as described above can be used as an anti-adhesion material. Anti-adhesion materials are defined as bioabsorbable synthetic materials directly used at an application area during surgery for the purpose of mitigating postoperative adhesion. ("Special treatment materials and material prices (standard material prices)" Heisei 28 Ministry of Health, Labour and Welfare notification No. 402). Anti-adhesion materials are generally called "anti-adhesion absorptive barriers."

The anti-adhesion material according to the present embodiment has the following advantages.

First, the anti-adhesion material according to the present embodiment, which is in a form of particles, can be more easily handled as compared with sheet-type anti-adhesion materials. Further, the anti-adhesion material (power) according to the present embodiment is a single-solution type, and does not require a cross-linking agent and the like. For this reason, it is less expensive and more easily handled as compared with a two-solution type. The anti-adhesion material according to the present embodiment can be applied to an affected area (defective area) using a general-purpose spray system. Therefore, it can be easily used not only in laparoscopic surgery but also in endoscopic surgery.

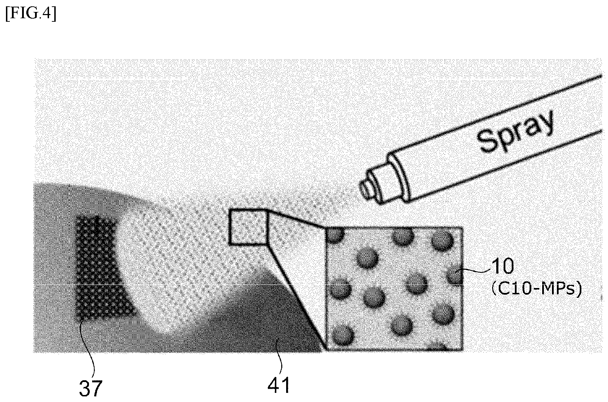

FIG. 4 shows a schematic diagram illustrating an application of the anti-adhesion material (particles) according to the present embodiment using a spray system. A defective area 37 is present at a tissue 41 in a living body. To this, particles 10 (for example, "C10-MPs") as an anti-adhesion material are applied using a spray device (indicated by "Spray" in the figure).

That is, the anti-adhesion material (particles) according to the present embodiment can be characterized by being "spray applicable" to the defective area 37.

The anti-adhesion material (particles) according to the present embodiment comprises a cross-linked product of a gelatin mixture. The gelatin mixture has a hydrocarbon group introduction ratio in a specific range as described above. As a result, the particles according to the present embodiment have appropriate hydrophobicity.

By virtue of this appropriate hydrophobicity, the particles applied on a tissue of an affected area during a surgical operation are hydrated, and, for example, are fused due to hydrophobic interactions between the particles to form a colloidal gel layer. This colloidal gel layer serves as a physical barrier to give anti-adhesion capability.

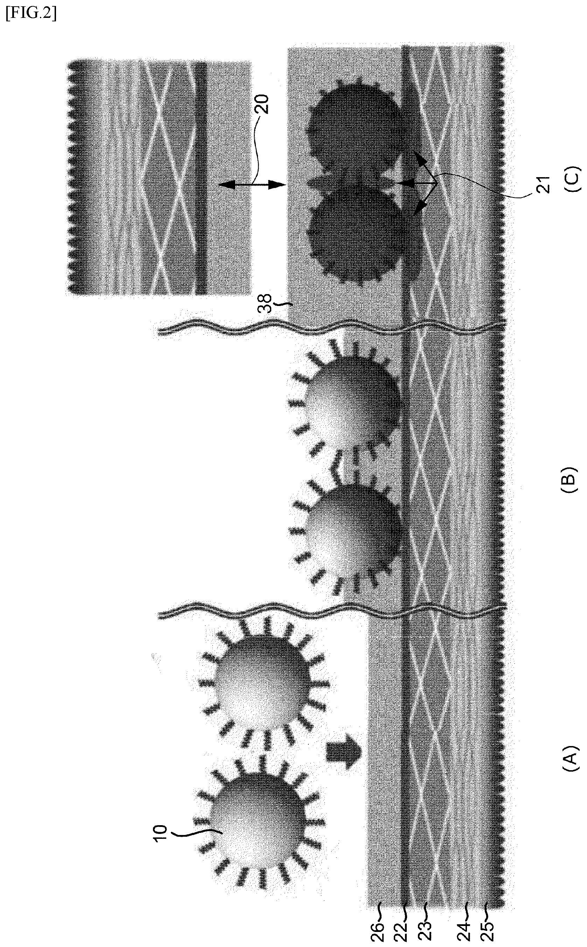

Fig. 2 is a schematic diagram illustrating a function of the anti-adhesion material (particles).

First, the particles 10 as the anti-adhesion material adhere to a tissue (A). The tissue has a laminate structure of a serosa layer 22, a muscle layer 23, a submucous layer 24, and a mucosa layer 25, and has a surface on which an aqueous layer 26 is present.

When adhering to the tissue, the particles 10 are gradually hydrated by the aqueous layer 26 (B). The particles 10 adhering to the surface of the tissue form a layer of a colloidal gel 38 (a colloidal gel layer) on the serosa layer 22 (C). The layer of the colloidal gel 38 strongly adheres to the serosa layer 22 by hydrophobic interactions 21. On the other hand, the colloidal gel 38 shows non-adhesiveness 20 with another tissue.

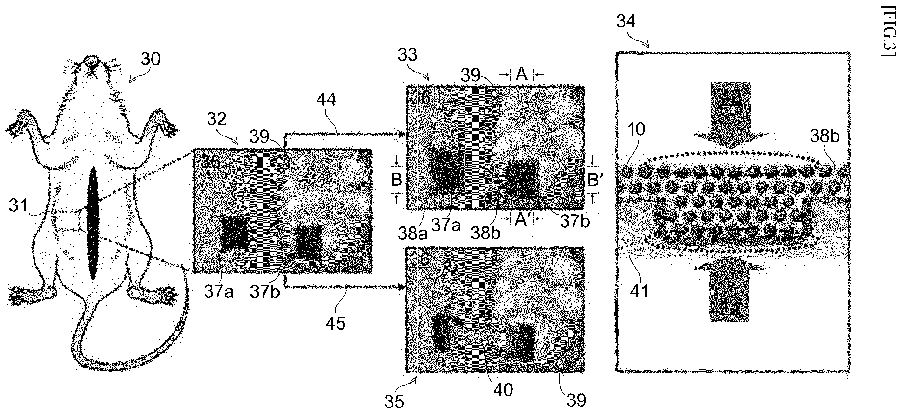

FIG. 3 shows a schematic diagram illustrating a function of the anti-adhesion material (particles) according to the present embodiment in a living body (rat). The defective areas 37 (37a, 37b) are present at an inside of the abdomen 31 (an abdominal cavity 36) and a cecum 39 of a rat 30, respectively. An image 32 represents a state before the treatment with the anti-adhesion material (the particles 10).

Next, the anti-adhesion material (the particles 10) is applied to the defective area 37 (an arrow 44). An image 33 represents a state after the anti-adhesion material (the particles 10) is applied. The particles 10 are applied to both of the defective areas 37a and 37b, and hydrated. Thereby, the layers of the colloidal gels 38a and 38b are formed on the defective areas 37a and 37b.

An image 34 shows a schematic diagram of a cross section of the tissue at the A-B portion. The layer of the colloidal gel 38b is formed on the tissue 41 with the hydrated particles 10. The layer of the colloidal gel 38b shows non-adhesiveness as a physical barrier against another tissue (an arrow 42). In contrast, it shows tissue adhesiveness due to hydrophobic interactions between the particles 10 and the tissue 41 at the side of the tissue 41 (an arrow 43). This can prevent adhesion between the tissue 41 and another tissue.

A case where the anti-adhesion material (the particles 10) is not used (an arrow 45) will be described. An image 35 represents a state where the anti-adhesion material (the particles 10) is not used. In this case, the defective areas 37a and 37b adhere each other to form an adhesion 40.

The colloidal gel layer of the particles having appropriate hydrophobicity according to the present invention absorbs water at the surface of the affected tissue, and forms hydrophobic interactions between the particles and the affected tissue, resulting in strong adhesion to the surface of the affected area. However, after complete hydration, the colloidal gel layer is no longer adhesive to another tissue due to the presence of water on the surface of the colloidal gel layer and on the surface of the tissue (the surface of another tissue, different from the tissue where the colloidal gel layer is formed). Thus, the anti-adhesion material (particles) according to the present embodiment has both adhesiveness to an affected tissue and non-adhesiveness (anti-adhesion capability) to other tissues (see FIGs 2 and 3). Conventional common anti-adhesion materials function as physical barriers, but they are less adhesive to soft tissues in moist conditions, showing a problem of easy detachment from tissue defective areas (affected areas). The anti-adhesion material according to the present embodiment can solve this problem as described above.

Anti-adhesion materials need to remain stable at an affected area under moist conditions for a certain period of time. If an anti-adhesion material does not stably adhere to an affected area under physiological conditions (moist conditions), postoperative adhesion may be induced by fibrin deposition and fibroblast migration. The anti-adhesion material having appropriate hydrophobicity according to the present invention forms hydrophobic interactions between a colloidal gel layer and an affected tissue, thereby showing high stability even in the moist conditions.

Digestive organs such as the stomach, the duodenum, and the large intestine contract frequently during peristaltic movement. Therefore, the followability of an anti-adhesion material is important on a tissue which changes its shape. For the anti-adhesion material according to the present invention, the colloidal gel layer adheres firmly to an affected tissue and shows excellent followability to a deforming tissue. Further, the anti-adhesion material according to the present embodiment may be characterized by that the colloidal gel layer is quickly decomposed and absorbed after the colloidal gel layer has completed its anti-adhesion function.

When an anti-adhesion material (particles) is produced from only one type of hydrophobized gelatin, a procedure for adjusting the hydrocarbon group introduction ratio to a specific value may be complicated. This is because the percentage of introduction of hydrocarbon groups needs to be adjusted in consideration of yield and other factors, which also needs to be measured for confirmation.

The anti-adhesion material (particles) according to the present invention has an advantage in this respect because a mixture of the raw material gelatin (the first gelatin) and the hydrophobized gelatin (the second gelatin) can be used.

The anti-adhesion material (particles) according to the present invention only requires adjusting a mixing ratio (A) of the raw material gelatin and the hydrophobized gelatin based on the hydrocarbon group introduction ratio (Xc) of the hydrophobized gelatin. Consequently, the hydrocarbon group introduction ratio (Xm) of the gelatin mixture, i.e., the hydrophobicity of the anti-adhesion material (particles) can be adjusted easily (see the Equation (I) above).

It is also possible to produce multiple types of anti-adhesion materials having different degrees of hydrophobicity from a single type of hydrophobized gelatin by changing the mixing ratio (A). A less amount of a hydrophobized gelatin may be required as compared with a case where an anti-adhesion material is produced from only one type of hydrophobized gelatin. This is because the anti-adhesion material according to the present embodiment is prepared by mixing the raw material gelatin with the hydrophobized gelatin.

The hydrophobized gelatin, which is synthesized from the raw material gelatin, requires more time and efforts for synthesis, and also suffers from a production cost higher than that of the raw material gelatin. The anti-adhesion material is also superior in that the amount of the hydrophobized gelatin required for producing particles having predetermined hydrophobicity tends to be relatively small. This is because time and efforts required for synthesis and the production cost depend not on the amount of the hydrophobized group introduced into the raw material gelatin, but on the amount of the hydrophobized gelatin to be synthesized and the number of times for synthesis. As a result, the synthesis requires less time and effort, enabling a reduced production cost.

The anti-adhesion material according to the present embodiment, which has the advantages as described above, is suitable for mass production in industrial settings. Mass production can further reduce a production cost.

The particles according to the present embodiment may be used as an anti-adhesion material, but can also be used as a wound dressing material at the same time. That is, they can be used as an article having two functions: wound dressing and anti-adhesion. For example, when applied to a postoperative damaged area, a film having a wound dressing effect and an anti-adhesion effect may be formed. Compared with the conventional separate application of a wound dressing material and an anti-adhesion material, the both effects can be achieved more conveniently. Further, the particles according to an embodiment of the present invention have excellent blood coagulation ability and can be used as a hemostatic material. In addition to the functions of wound dressing and anti-adhesion, it can also be used to form an article having blood coagulation ability.

### <Method of manufacturing particles>

There is no particular limitation for the method of manufacturing the particles according to the present embodiment. For example, they may be manufactured by using the coacervation method disclosed in Patent Document 1. Below, an example of the method of manufacturing the particles according the present embodiment will be described.

The method of manufacture according to the present embodiment comprises, for example, the following steps.
Step 1: a step of dissolving a gelatin mixture of a raw material gelatin and a hydrophobized gelatin in a good solvent to prepare a gelatin mixture solution,
Step 2: a step of adding a poor solvent to the gelatin mixture solution, precipitating a first intermediate particle containing the gelatin mixture in the gelatin mixture solution,
Step 3: a step of lyophilizing the gelatin mixture solution including the first intermediate particle to obtain a second intermediate particle,
Step 4: a step of cross-linking the gelatin mixture of the second intermediate particle to obtain a particle having the gelatin mixture cross-linked.

### Step 1:

The step 1 is a step of dissolving the gelatin mixture as already described in a good solvent to obtain a gelatin mixture solution. As used herein, the term "good solvent" means a solvent in which the gelatin mixture can easily be dissolved. There is no particular limitation for the type of the good solvent, but examples include water, glycerin, acetic acid, a mixture thereof, and the like. Among them, it preferably contains water. The above good solvent may be heated. There is no particular limitation for a heating temperature, but it is preferably 50°C to 70°C.

There is no particular limitation for a method of dissolving the gelatin mixture in a good solvent, but a publicly known method can be used. For example, the followings may be used: a method comprising adding a good solvent at low temperature (e.g., room temperature) to a gelatin mixture to swell the gelatin mixture, and heating the resulting swollen material to obtain a gelatin mixture solution (a swelling and dissolution method); and a method comprising charging a gelatin mixture into the above good solvent which is preheated to obtain a gelatin mixture solution (a direct dissolution method).

There is no particular limitation for the content of the gelatin mixture in the gelatin mixture solution, but the content of the gelatin mixture (the final concentration) is preferably 0.01 mass/vol% to 30 mass/vol%, more preferably 1 mass/vol% to 25 mass/vol%, even more preferably 5 mass/vol% to 20 mass/vol%, and in particular preferably 5 mass/vol% to 15 mass/vol% relative to the total volume of the gelatin mixture solution.

### Step 2:

The step 2 is a step of adding a poor solvent to the gelatin mixture solution to precipitate a first intermediate particle containing the gelatin mixture in the gelatin mixture solution (coacervation). As used herein, the term "poor solvent" means a solvent in which the gelatin mixture is more difficult to be dissolved as compared with in the good solvent used in the step 1. That is, good and poor solvents are not defined by the absolute solubility of the gelatin mixture, but defined relatively with respect to the relationship between a poor solvent and a good solvent.

There is no particular limitation for the poor solvent, but examples include organic solvents, and it is preferably, among them, a water-miscible organic solvent, more preferably alcohols, for example, methanol, ethanol, propanol, isopropanol, butanol, t-butyl alcohol, and the like.

When a poor solvent is added to the gelatin mixture solution, the first intermediate particle precipitates in the gelatin mixture solution. The first intermediate particle is a particulate material containing the above gelatin mixture. There is no particular limitation for the particle diameter of the first intermediate particle precipitated in this step, but it is preferably 0.1 µm to 100 µm, more preferably 1 µm to 50 µm, and even more preferably 1 µm to 10 µm. If the particle diameter is in the above ranges, sedimentation of the intermediate particle which precipitated in the gelatin mixture solution is more difficult. As a result, during a process in the step 3 of lyophilization described below, aggregation of the intermediate particles can be better prevented.

There is no particular limitation for a temperature at the time of adding the poor solvent, but it is preferably 10°C to 30°C, more preferably 15°C to 25°C. In the step 1, when the solvent is heated to dissolve the gelatin mixture, it is preferred to further have a step of cooling the gelatin mixture solution between the step 1 and the step 2.

When the poor solvent is added dropwise, it is preferred to agitate the gelatin mixture solution. There is no particular limitation for a method of agitation, but a publicly known method can be used. By adding the poor solvent to the gelatin mixture solution with agitating, the precipitated particles are less likely to aggregate and less likely to settle down.

### Step 3:

The step 3 is a step of lyophilizing a dispersed solution of uncross-linked gelatin particle precipitated by the above coacervation (the first intermediate particle) to obtain the second intermediate particle. There is no particular limitation a method of freezing the gelatin mixture solution, but more rapid freezing is preferred. This enables the particles containing the uncross-linked gelatin mixture to be less likely to aggregate upon freezing.

There is no particular limitation for the atmosphere temperature upon freezing, it is preferably -20°C or less, more preferably -30°C or less. There is also no particular limitation for a method of lyophilization, but a publicly known method can be used. The second intermediate particle includes the first intermediate particle. The second intermediate particle may also include a component other than the first intermediate particle. Examples of such a component include, for example, the good solvent and the poor solvent described above, and the like.

### Step 4:

The step 4 is a step of cross-linking the gelatin mixture of the second intermediate particle to obtain a particle containing the cross-linked gelatin mixture (the cross-linked product). The gelatin mixture of the particle irreversibly undergoes inter- and/or intramolecular cross-linking in this step. As a result, the particle containing the cross-linked product of the gelatin mixture can be obtained.

There is no particular limitation for the cross-linking method, but examples of the method include a method comprising applying thermal energy to the gelatin mixture or irradiating the gelatin mixture with an active ray or radiation (e.g., an electron beam and the like). Among these, a method (thermal cross-linking) comprising applying thermal energy (in other words, heating) is preferred because it can more easily produce the cross-linked product of the gelatin mixture, and it is safe in terms of generation of no impurities from a cross-linking agent. In this method, an amino group reacts with other reactive groups (for example, a carboxy group, a mercapto group, and the like) in the gelatin mixture to form a cross-linked structure.

There is no particular limitation for a method of thermal cross-linking, but a publicly known method can be used. Examples of the method of thermal cross-linking include a method comprising placing a entire container with the second intermediate particles in a heated atmosphere (e.g., in an oven) and maintaining it for a predetermined time.

There is no particular limitation for the heating temperature in the thermal cross-linking, but it is, in general, preferably 80°C to 200°C, more preferably 100°C to 200°C. There is no particular limitation for the heat time in the thermal cross-linking, but it is, in general, preferably 0.1 hour to 20 hours, more preferably 0.5 hour to 10 hours, even more preferably 1 hour to 6 hours, still more preferably 2 hours to 5 hours, and in particular preferably 2.5 hours to 4 hours. If the heating time is within the above numerical ranges, the resulting particles tend to have superior adhesiveness.

The cross-linked product of the gelatin mixture may be obtained by allowing the gelatin mixture to react with a cross-linking agent. There is no particular limitation for the cross-linking agent, but examples include polybasic acids, aldehyde compounds, acid anhydrides, dithiocarbonates, di-isothiocyanates activated by genipin, N-hydroxysuccinimide, N-sulfoxysuccinimide, and the like. As the cross-linking agent, for example, compounds described in the paragraphs 0021 to 0024 of WO2018/079538 can also be used.

### EXAMPLES

The present invention will be described in more detail with reference to Example below. Materials, usage amounts, percentages, treatment details, treatment procedures, and the like which are shown in Example below can be appropriately altered without departing the spirits of the present invention. Therefore, the scope of the present invention should not be construed as limited to Example as shown below.

### 1. Production of Particles

### [Synthesis of hydrophobized gelatin]

A gelatin derived from Alaska pollock (hereinafter may be referred to as "Org-ApGltn" or simply "Org" as appropriate) was used as the raw material gelatin. A gelatin derivative (hereinafter may be referred to as "C10-ApGltn" as appropriate) in which a decyl group was introduced to Org-ApGltn was used as the hydrophobized gelatin. The hydrophobized gelatin was obtained by allowing decanal to react with an amino group in the raw material gelatin to form a Schiff base, and then reducing the resulting Schiff base into a stable secondary amine with a reducing agent. Below, the synthesis of the hydrophobized gelatin are described in detail.

A gelatin from Alaska pollock (Org-ApGltn) (from Nitta Gelatin Inc.; weight average molecular weight (Mw): 38,552 Da; the content of amino groups: 339 µmol/g) in an amount of 100 g was dissolved in 105 mL of ultrapure water. The resulting solution was heated to 50°C, and two equivalents of decanal (Tokyo Chemical Industry Co., Ltd., 67.8 mmol) with respect to the amino groups (339 µmol/g) of Org-ApGltn was added along with ethanol (Junsei Chemical Co., Ltd.). By this, an imine bond was formed between the amino groups of decanal and Org-ApGltn.

After agitating the above solution at the same temperature (50°C) for 1 hour, 2-picoline borane (Junsei Chemical Co., Ltd., 50.85 mmol) was added along with ethanol to reduce the imine. The resulting mixed solution (the concentration of Org-ApGltn: 20 mass/vol%; water:ethanol = 105:45 mL) was agitated at 50°C for 17 hours to proceed the reaction.

The mixed solution (150 mL) after the reaction was added dropwise to 1500 mL of cold ethanol (-7 to 4°C) to purify C10-ApGltn. The resulting re-precipitated material was washed (1 hour × 3 times) with 1500 mL of ethanol to remove unreacted decanal and 2-picoline borane. Subsequently, it was dried in a vacuum for 3 days to obtain C10-ApGltn at a yield of 92.9 mass%.

The introduction of decyl groups into the resulting C10-ApGltn was able to be confirmed by Fourier transform infrared spectroscopy and ¹H-NMR. The hydrocarbon group introduction ratio of C10-ApGltn (hereinafter may be referred to as the "decyl group introduction ratio DS" or simply "DS" as appropriate) was computed. DS was computed from the number of amino groups in the raw material gelatin and the number of amino groups in the hydrophobized gelatin quantified by the 2,4,6-trinitrobenzenesulfonic acid method (the TNBS method).

The computed DS of C10-ApGltn was 49 mol%. This means that 6.4 decyl groups are introduced into amino groups in a single molecule of C10-ApGltn. Below, a hydrophobized gelatin having a DS of 49 mol% may be denoted as 49C10-ApGltn (or simply "49C10"). The DS values of the resulting 49C10-ApGltn are summarized in Table 1.

**[Table 1]**

| Abbreviation | ApGltn | Theoretical DS | DS | Yield |
|---|---|---|---|---|
| | (g) | (mol%) | (mol%) | (Mass%) |
| 49C10-ApGltn | 100 | 200 | 49 | 92.9 |

### [Production of particles]

By the coacervation method using ethanol as a poor solvent for a hydrophobized gelatin, 8 different types of particles having different hydrophobicities were produced with the raw material gelatins and/or the hydrophobized gelatins in the ratios as shown in Table 2 (see FIG. 1). Below, the production of particles will be described in detail.

### <Sample 1 (49C10-MPs)>

First, the synthesized 49C10-ApGltn was dissolved in ultrapure water at 50°C to obtain a 5 mass/vol% aqueous solution of C10-ApGltn. Next, aliquots of ethanol were added dropwise to the solution in small quantities with agitating (400 rpm) at room temperature (25°C). The resulting solution was preliminarily frozen at -30°C for 24 hours and then lyophilized. The resulting dried particles were thermally cross-linked by heating under vacuum conditions (less than 3 mbar) at 150°C for 3 hours to form an amide bond between a carboxy group and an amino group in 49C10-ApGltn. This yielded particles (49C10-MPs) as a cross-linked product of only 49C10-ApGltn.

### <Sample 2 (Org-MPs)>

The sample 2 was produced as in the sample 1, except that the raw material gelatin (Org-ApGltn) was used instead of the hydrophobized gelatin (49C10-ApGltn).

### <Samples 3 to 8>

The hydrophobized gelatin (49C10-ApGltn) was used alone in the sample 1, but mixtures of the raw material gelatin and the hydrophobized gelatin in the mixing ratios as shown in Table 2 was used in the sample 3 to 8 instead. Except for this, the samples 3 to 8 (10C10-MPs to 45C10-MPs) were produced as in the sample 1.

The yields of the samples 1 to 8 (particles) and the DSs of the raw material of the particles (the raw material gelatin, the hydrophobized gelatin, or the gelatin mixture) are also shown in Table 2. DSs were computed by the equation (I) as described above.

**[Table 2]**

| Sample No. | Abbreviation | Org-ApGitn mass | 49C10-ApGltn mass | DS | Total mass | Yield |
|---|---|---|---|---|---|---|
| | | (9) | (9) | (mol%) | (g) | (mass%) |
| 2 | Org-MPs | 5.00 | 0 | 0 | 5.00 | 84.1 |
| 3 | 10C10-MPs | 3.98 | 1.02 | 10 | 5.00 | 82.5 |
| 4 | 20C10-MPs | 2.96 | 2.04 | 20 | 5.00 | 84.4 |
| 5 | 30C10-MPs | 1.94 | 3.06 | 30 | 5.00 | 83.3 |
| 6 | 35C10-MPs | 1.43 | 3.57 | 35 | 5.00 | 84.3 |
| 7 | 40C10-MPs | 0.92 | 4.08 | 40 | 5.00 | 80.9 |
| 8 | 45C10-MPs | 0.41 | 4.59 | 45 | 5.00 | 84.3 |
| 1 | 49C10-MPs | 0 | 5.00 | 49 | 5.00 | 85.3 |

### 2. Evaluation

### [SEM observation of particles and particle diameter distribution]

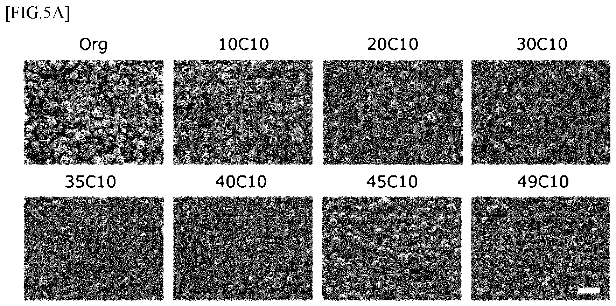

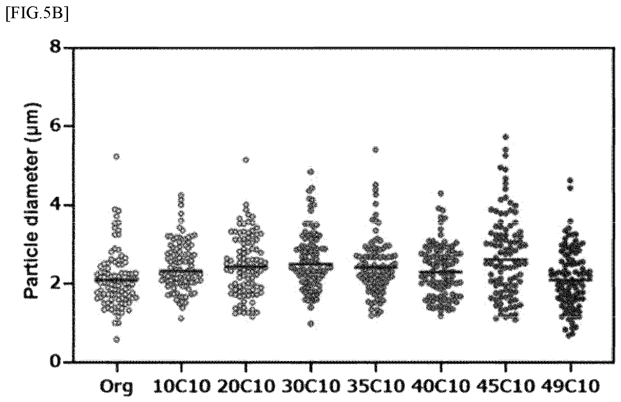

The resulting samples 1 to 8 was observed by SEM, and particle diameter distribution was analyzed with "Image J." As shown in FIG. 5A, all of the samples 1 to 8 were spheres (microparticles) having microscale particle diameters. Moreover, as shown in FIG. 5B, the average particle diameters of the samples 1 to 8 were 2 to 3 µm, and the particle diameter distribution of each sample did not change with DS.

It is noted that "Org" in FIGs. 5A and 5B represents "Org-MPs" of the sample 2. Similarly, "10C10" represents "10C10-MPs" of the sample 3. In addition, "20C10," "30C10," "35C10," "40C10," "45C10," and "49C10" represent the samples 4, 5, 6, 7, 8, and 1, respectively.

### [Spray tests]

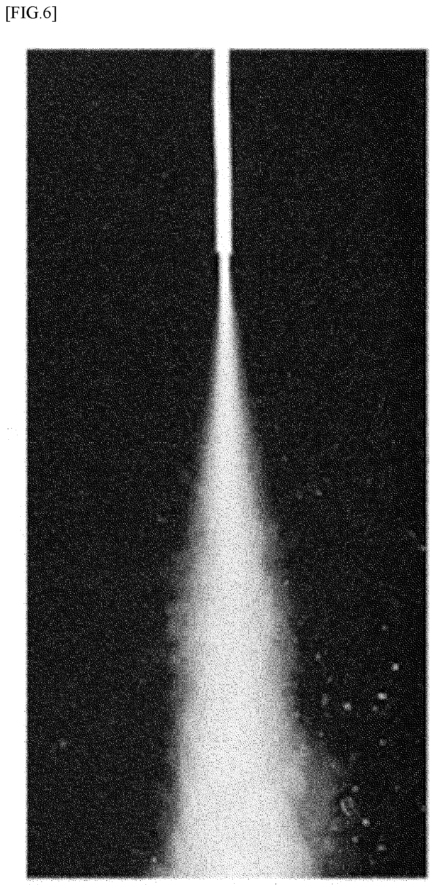

The samples 1 to 8 produced were subjected to spray tests using an endoscopic spray device (the spray system). As shown in FIG. 6, the samples 1 to 8 were sprayable with the endoscopic spray device, confirming that they were directly sprayable to, for example, a surface of an internal organ tissue during surgery.

### [Production and evaluation of colloidal gel]

### <Water content of colloidal gel>

The sample 8 (45C10-MPs) in an amount of 50 mg was packed into a silicon mold with a thick of 1 mm and a diameter of 10 mm and leveled using a spatula. A colloidal gel was produced by adding dropwise 300 µL of physiological saline (Otsuka Pharmaceutical Co., Ltd.) to allow hydration for a predetermined time (hydration time: 1 to 30 minutes). Excess water on the surface of the colloidal gel was then removed, and the resulting colloidal gel was weighed (Ww) and lyophilized. Finally, the weight (Wd) of the gel after dried was measured, and the water content of each colloidal gel was computed by the following equation.$Water content \left(%\right) = \frac{{W}_{w} - {W}_{d}}{{W}_{w}} \times 100$

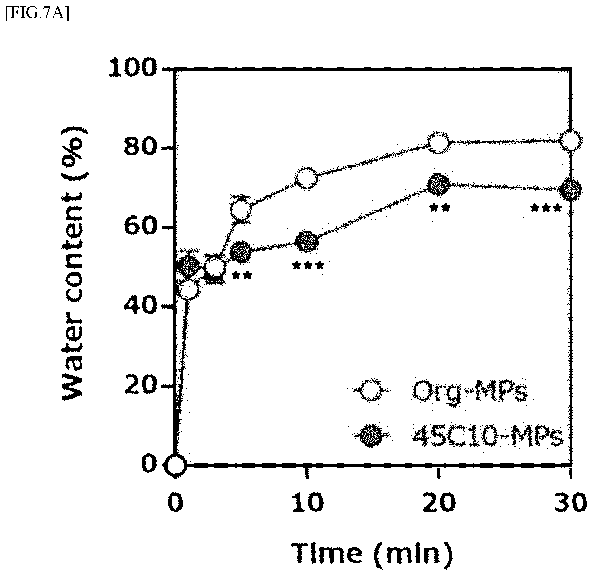

FIG. 7A shows the time dependent water content of a colloidal gel (the relationship between hydration time and water content) for sample 8 (45C10-MPs). The time dependent water content of a colloidal gel was also evaluated for the sample 2 (Org-MPs) in a similar manner. The results are shown together in FIG. 7A.

As can be understood from FIG. 7A, the sample 2 (Org-MPs) and the sample 8 (45C10-MPs) gradually absorbed water and formed colloidal gels, reaching the equilibrium of swelling after 30 minutes. In terms of the water content at 5 minutes or later, the water content (53 to 69%) of the sample 8 (45C10-MP) with higher DS was lower than that of the sample 2 (Org-MPs) (64 to 82%), confirming that the sample 8 has higher hydrophobicity.

It is noted that the tests were performed with n = 3. In FIG. 7A, "**" means P < 0.01 and "***" means P < 0.001.

### <Observation of particle fusion>

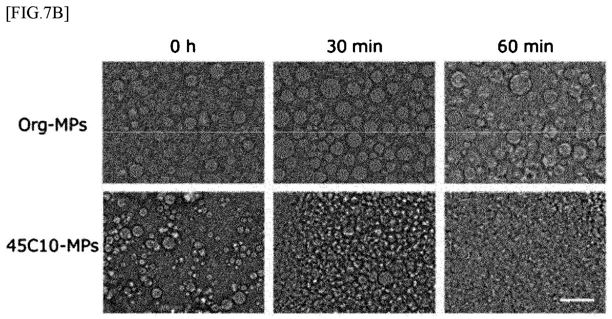

Particles were observed for their aggregation behavior under moist conditions. The sample 2 (Org-MPs) and the sample 8 (45C10-MPs) each in an amount of 10 mg were added to 200 µL of physiological saline to prepare suspensions. After incubating these suspensions for up to 2 hours, the particles were observed for their aggregation behavior under a bright field microscope (KEYENCE CORPORATION, BZ-X710). Bright field micrographs are shown in FIG. 7B.

Org-MPs was isolated from each other at any hydration time, and fusion of particles was not able to be observed. In contrast, 45C10-MPs aggregated over hydration time and formed a colloidal gel due to hydrophobic interactions. 45C10-MPs showed a tightly packed structure after 30 minutes of hydration. Almost all 45C10-MP were then fused with each other to form large assemblies after hydration for 60 minutes. These results also suggest that hydrophobic interactions between 45C10-MPs are the driving force for formation of a colloidal gel under moist conditions.

### <in vitro tissue adhesion/non-adhesion tests>

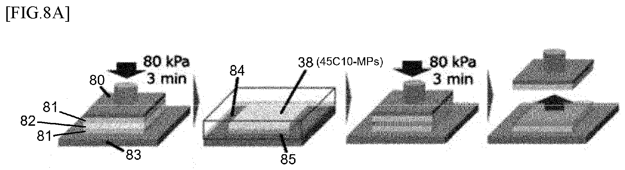

An impact of hydration time on adhesive strength of a colloidal gel to tissue was evaluated. The tests were performed by a method in accordance with the standard (ASTM F-2258-05) by the American Society for Testing and Materials (see FIG. 8A).

First, fresh porcine stomach (purchased from Tokyo Shibaura Zouki Co.,Ltd.) was opened, and the mucosa layer was removed to expose a serosa tissue (81) which is a submucous tissue. The resulting serosa tissue (81) was cut into tissue pieces of 2.5 cm squared, which were then fixed on each stage of the upper and lower jigs (80) of a test apparatus (a texture analyzer TA-XT2i from Stable Micro Systems) with a cyanoacrylate glue (Henkel Japan Ltd.). A hot plate (83) was used to maintain the temperature of the porcine stomach lining tissue at 37°C during the measurements.

In order to remove excess surface water on the above tissue, an industrial paper rag (82) (under the trade name of "Kimwipe") was pressed against the surfaces at 80 kPa for 3 minutes to remove water. Then, 100 mg of the sample 8 (45C10-MPs) was sprinkled so as to cover the tissue on the stage of the lower jig and hydrated for a predetermined time (hydration time: 1 to 30 minutes) under moist environments. This resulted in formulation of the layer of the colloidal gel (38). Specifically, physiological saline (84) (7 mL) was filled around the tissue to bring the humidity around the tissue surfaces and the sample 8 (45C10-MPs) closer to that of the intestinal environment, and a plastic cover (85) was used to cover the tissue.

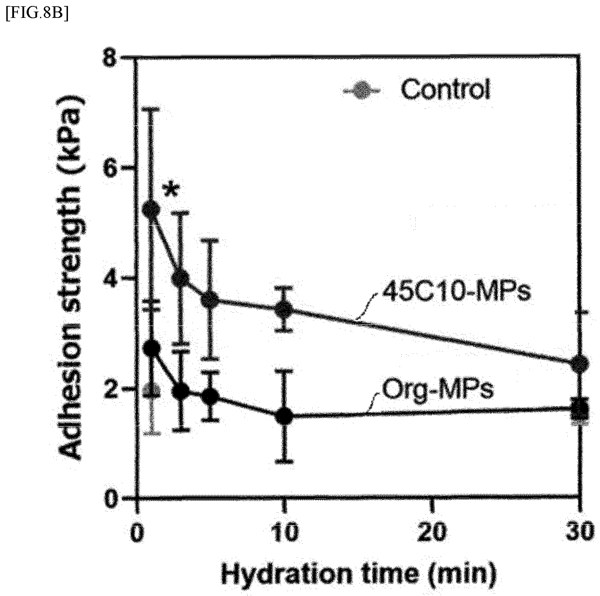

After hydration for a predetermined period of time, the upper jig was placed over the sample 8 (the colloidal gel 38) on the lower jig, and crimped for 3 minutes under a pressure of 80 kPa. The upper jig was then lifted up at 10 mm/min, and the adhesive strength of the sample 8 (45C10-MPs) was measured. FIG. 8B shows the relationship between hydration time and adhesive strength. The sample 2 (Org-MPs) was also evaluated in a similar way, and results are shown in FIG. 8B. As a control, the adhesive strength between two tissues that were not sprayed with the particles is also shown in FIG. 8B.

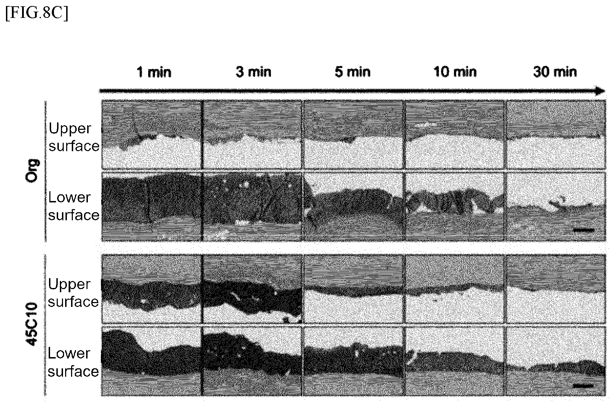

Further, after measuring adhesive strength, tissue sections were fixed with 10% formalin buffer (Wako Pure Chemical Industries, Ltd.) and stained with hematoxylin-eosin (HE). The interface between the colloidal gel and the tissue was then observed. Bright field micrographs are shown in FIG. 8C.

As shown in FIG. 8B, the adhesive strength of the colloidal gel of the sample 2 (Org-MPs) did not change significantly as hydration time increased (the maximum strength: 2.73 kPa, the minimum strength: 1.48 kPa). In contrast, the adhesive strength of the colloidal gel of the sample 8 (45C10-MPs) decreased from 5.24 kPa to 2.41 kPa with the hydration time, and dropped to around the adhesive strength of the colloidal gel of the sample 2 (Org-MPs) after 30 minutes of hydration.

As can be understood from the observation of HE staining shown in Figure 8C, the colloidal gel of the sample 8 (45C10-MPs) adhered to both of the upper surface and the lower surface of the tissue after 1 minute of hydration and 3 minutes of hydration while the amount of the colloidal gel adhered to the upper surface of the tissue was reduced after 30 minutes of hydration. The results show that the sample 8 (45C10-MPs) is fully hydrated to form a colloidal gel in 30 minutes of hydration time and at the same time becomes non-adhesive to other tissues. During the process of hydration of particles, the sample 8 (45C10-MPs) can absorb water from the tissue surfaces and form hydrophobic interactions between the particles and the tissue, resulting in strong adhesion. Then, after complete hydration, the colloidal gel layer is considered to be no longer adhesive (showing anti-adhesion effects) due to the presence of water on the surfaces of the colloidal gel and the tissue (see FIG. 2).

### [Underwater stability tests]

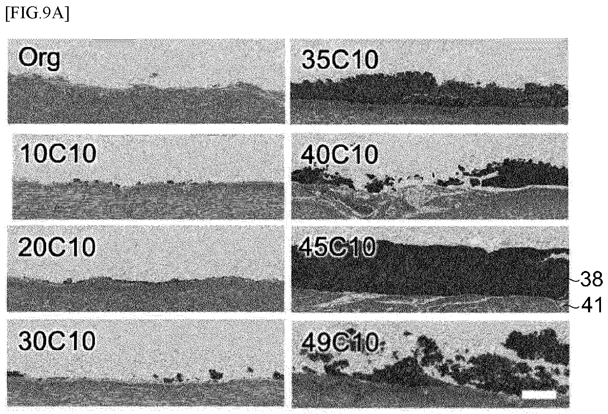

The underwater stability of the colloidal gel of the particles was evaluated by immersion in physiological saline after the colloidal gel was allowed to adhere to the stomach tissue (the submucosal layer). First, a porcine stomach tissue was cut into 2.5 × 2.5 cm pieces. The sample 8 (45C10-MPs) in an amount of 50 mg was sprinkled roundly in a 10 mm diameter on the tissue and hydrated with 300 µL of physiological saline for 30 minutes to form a colloidal gel. The colloidal gel adhered to the stomach tissue was immersed into physiological saline containing 0.05 mass/vol% of sodium azide (Wako Pure Chemical Industries, Ltd.), and incubated at 37°C for two days. Then, the colloidal gel adhered to the stomach tissue was fixed with 10% formalin buffer and the interface between the colloidal gel and the tissue was observed after HE staining. Bright field micrographs are shown in FIG. 9A. Similar evaluation was performed for the samples 1 to 7, and results are shown in FIG. 9A.

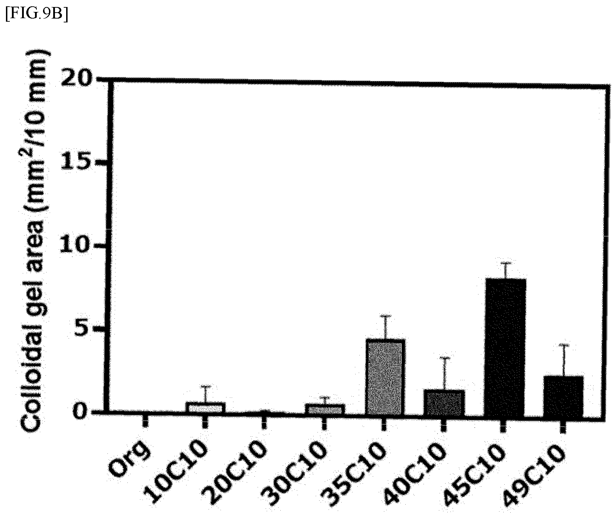

Further, image analysis of HE-stained images quantified the remaining area of the colloidal gel on the stomach tissue. The results from the samples 1 to 8 are shown in FIG. 9B. It is noted that in FIGs. 9A and 9B, each sample is represented by the first half part (a part before -MPs) of the corresponding particle name. For example, the particles 45C10-MPs (the sample 8) are designated as "45C10."

As shown in FIGs. 9A and 9B, no colloidal gel was observed on the surface of the tissue after the tests for underwater stability for the samples having a DS of less than 30 mol%. In contrast, the colloidal gels of the samples having a DS of 30 mol% or more, more preferably the colloidal gels of the samples having a DS of 35 mol% or more showed high underwater stability even after 2 days of immersion in physiological saline. The area of the colloidal gel (38) remaining on the surface of the tissue (41) tended to increase with DS increases, and increased hydrophobicity of the particles improved the stability of the colloidal gel on the tissue. Our previous studies (e.g., Acta Biomaterialia 99 (2019) 387-396) revealed that hydrophobic groups introduced into a gelatin can interact with extracellular proteins such as fibronectin and cells through hydrophobic interactions. Further, the stomach tissue is known to be composed mainly of collagen, and thus it may also be speculated that decyl groups interacted with hydrophobic amino acid residues in the collagen. From the above results and findings, it can be inferred that the particles produced in Example interacted with extracellular proteins and cells on the stomach tissue, leading to high underwater stability.

### [in vivo anti-adhesion tests]

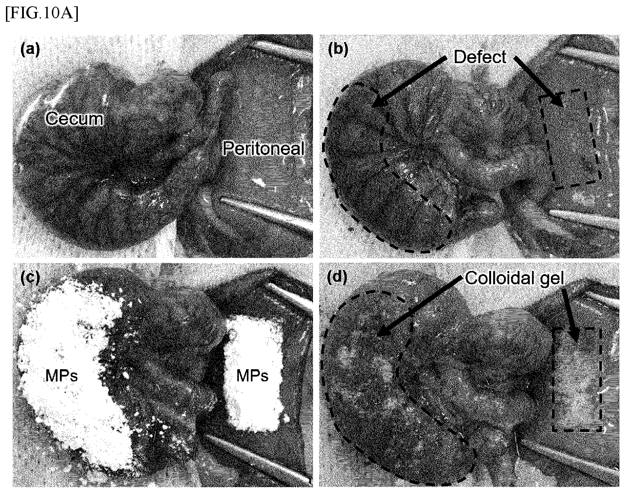

First, an abdominal wall-cecum defect model of SD (Sprague-Dawley) rats (purchased from Jackson Laboratory Japan, Inc., 7 weeks old male) was created as follows. A rat was anesthetized by introduction of 2.5% isoflurane, and abdominal hair was shaved, and ethanol was used to prevent infection. The abdomen was opened about 5 cm to expose the abdominal wall and the cecum ((a) in FIG. 10A). Cecum abrasion (cecum defective area) was created by rubbing the cecum with sterile gauze. An abdominal wall defect was created by excising an abdominal wall tissue (1 cm × 2 cm, including the mesothelial layer) at a location where it abuts the cecum defective area using a surgical knife ((b) in FIG. 10A).

Next, 100 mg of the sample 8 (45C10-MPs) which was heat sterilized at 150°C for 3 hours was sprinkled over each defective area to cover the wound area ((c) in FIG. 10A), and allowed to be hydrated with physiological saline for 15 minutes to form a colloidal gel ((d) in FIG. 10A). In order to prevent infection, amikamycin (1.0 mg/kg) was injected intraperitoneally, and the abdomen was then sutured and closed.

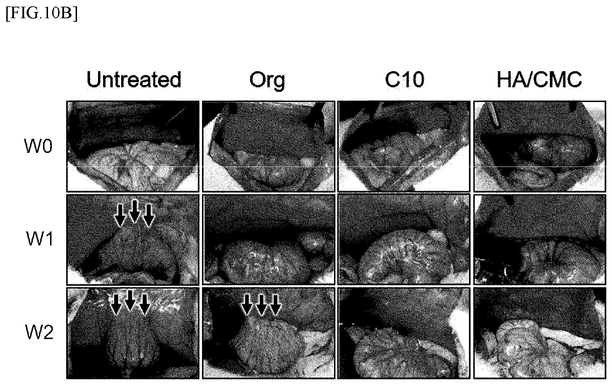

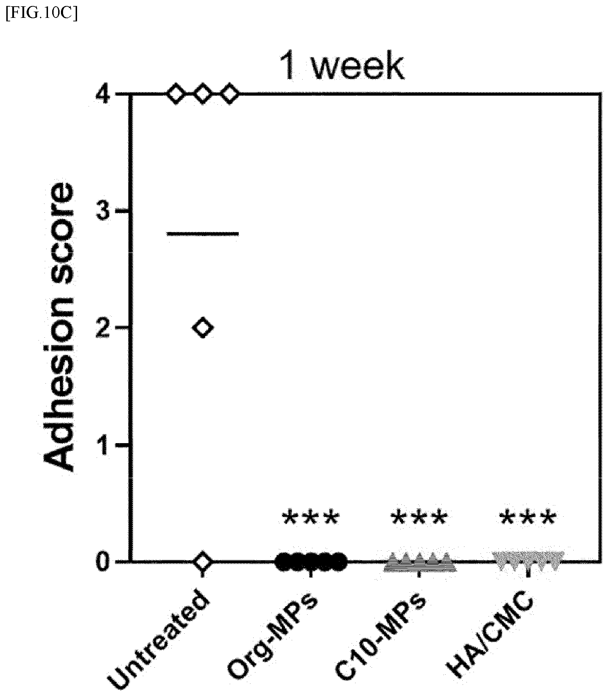

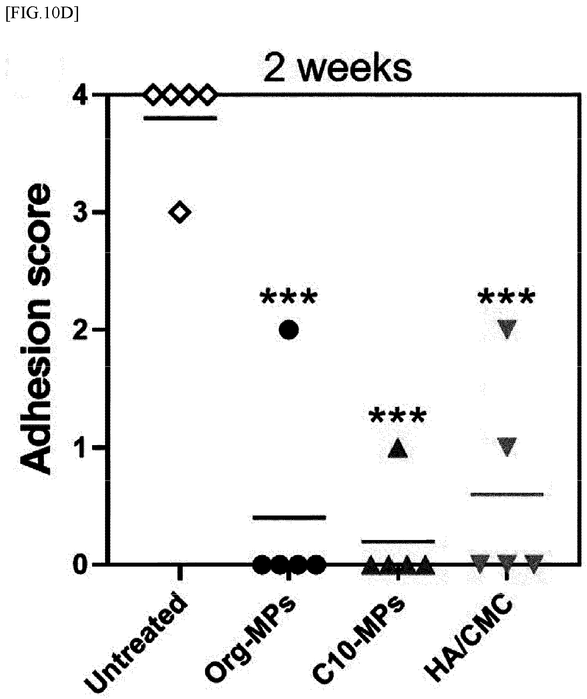

After a predetermined time (1 to 2 weeks), the abdomen was reopened, and postoperative adhesion was evaluated using the peritoneal wall adhesion scores (5 levels) as shown in Table 3. Results are shown in FIGs. 10C and 10D. A lower score indicates less adhesion. FIG. 10B shows photographs of the area around the cecum and peritoneal immediately before abdominal closure (W0), one week after abdominal closure (W1), and two weeks after abdominal closure (W2). Black arrows in the photographs indicate an adhesion area between the abdominal wall and the cecum. It is noted that the sample 8 (45C10-MPs) was indicated as "C10" in FIG. 10B, and as "C10-MPs" in FIGs. 10C and 10D. It is noted that the tests were conducted with n = 5, and *** represents P < 0.001.

**[Table 3]**

| Score | Peritoneal adhesion |
|---|---|
| 0 | No adhesion |
| 1 | Mild adhesions |
| 2 | Localized moderate adhesions |
| 3 | Moderate and wide adhesions |
| 4 | Severe adhesions, impossible to separate |

In place of the sample 8 (45C10-MPs), the sample 2 (Org-MPs) and a hyaluronic acid/carboxymethylcellulose sheet (HA/CMC) (a commercially available anti-adhesion material, Seprafilm^{®} from Kaken Pharmaceutical Co. Ltd.) were each used to perform the evaluation in the same way. Untreated rats that were not given the anti-adhesion material (Untreated) were also evaluated in the same way. These evaluation results are shown together in FIGs. 10B to 10D. It is noted that the sample 2 (Org-MPs) was indicated as "Org" in FIG. 10B.

After one week, severe adhesions (the average score: 2.80) were observed in the untreated rat group (Untreated) (Figure 10C), and adhesion layers were formed between the cecum and the abdominal wall (FIG 10B). This can be explained if the lack of a physical barrier between the tissues allowed for excessive fibrin deposition and migration of macrophages and fibroblasts, and fibrous tissues (FT) were formed between the cecum and peritoneum, resulting in formation of postoperative adhesion. In contrast, no adhesion was observed in the groups treated with the sample 8 (45C10-MPs) and the sample 2 (Org-MPs) and the commercially available HA/CMC sheet (FIG 10B, the average score: 0 for all the groups). These results may be due to the fact that the colloidal gel and the commercially available sheet effectively prevented migration of fibrin and inflammatory cells.

After two weeks, severe adhesions were observed in the untreated rat group (Untreated) with higher scores than those after one week (FIG. 10C, FIG. 10D). The other groups showed a slight increase in adhesion scores as compared to those after one week. Increased adhesion scores (scores: 2 and 3) were observed in the HA/CMC-treated group, consistent with the previous findings. In the sample 2 (Org-MPs)-treated group, adhesions (score: 2) were observed in one out of every five animals while only one animal in the sample 8 (45C10-MPs)-treated group showed mild adhesion.

The results of the anti-adhesion tests as described above were able to demonstrate that the sample 8 (45C10-MPs) adhered to the cecal abrasion and the peritoneal defective area after hydration to form a colloidal gel layer, and the colloidal gel layer functioned as a physical barrier to prevent postoperative adhesion after hydration, leading to prevention of postoperative adhesion.

### Industrial Applicability

The present invention can provide an anti-adhesion material which is excellent in adhesiveness on tissues and adhesion stability under aqueous environments, and has simple operability, and, in addition, can be easily mass produced in industrial settings.

### Reference Signs List

10 particles; 22 serosal layer; 23 muscle layer; 24 submucosal layer; 25 mucosa layer; 26 aqueous layer; 30 rat; 31 abdomen; 36 abdominal cavity; 37, 37a, 37b defective area; 38, 38a, 38b colloidal gel; 39 cecum; 40 adhesion; 41 tissue; 80 jig; 81 serosal tissue; 82 industrial paper rag; 83 hot plate; 84 physiological saline; 85 plastic cover.

## Claims

1. An anti-adhesion material comprising a particle,
the particle including a cross-linked product of a gelatin mixture of a first gelatin and a second gelatin having a hydrocarbon group introduced into the first gelatin,
a hydrocarbon group introduction ratio of the gelatin mixture being 30 mol% to 50 mol%,
the second gelatin having a structure represented by a following formula (1):
GltnNH-L-CHR¹R² (1)
wherein in the formula (1), Gltn represents a residue of gelatin, and L represents a single bond or a divalent linking group, and R¹ represents an alkyl group having 1 to 20 carbon atoms, and R² represents a hydrogen atom or an alkyl group having 1 to 20 carbon atoms.

2. The anti-adhesion material according to claim 1, wherein L is a single bond or - C(O)- in the formula (1).

3. The anti-adhesion material according to claim 2, wherein L is a single bond in the formula (1).

4. The anti-adhesion material according to any one of claims 1 to 3, wherein R¹ represents a linear alkyl group, and R² represents a hydrogen atom in the formula (1).

5. The anti-adhesion material according to any one of claims 1 to 4, wherein the hydrocarbon group introduction ratio of the second gelatin is higher than that of the gelatin mixture.

6. The anti-adhesion material according to any one of claims 1 to 5, wherein the hydrocarbon group introduction ratio of the second gelatin is 35 mol% to 80 mol%.

7. The anti-adhesion material according to any one of claims 1 to 6, wherein the hydrocarbon group introduction ratio of the gelatin mixture is 35 mol% to 45 mol%.

8. The anti-adhesion material according to any one of claims 1 to 7, wherein a percentage of a mass of the second gelatin to a total mass of the first gelatin and the second gelatin is 35 mass% to 90 mass%.

9. The anti-adhesion material according to any one of claims 1 to 8, wherein the first gelatin is an alkali-treated gelatin.

10. The anti-adhesion material according to any one of claims 1 to 9, wherein the first gelatin is a gelatin with a low endotoxin content.

11. The anti-adhesion material according to any one of claims 1 to 10, wherein the first gelatin is derived from cold-water fish.

12. The anti-adhesion material according to any one of claims 1 to 11, which is applicable to an affected area in a living body with a spray system.

13. A method of manufacturing the anti-adhesion material according to any one of claims 1 to 12, the method comprising:
dissolving the gelatin mixture of the first gelatin and the second gelatin in a good solvent to prepare a gelatin mixture solution;
adding a poor solvent to the gelatin mixture solution to precipitate a first intermediate particle containing the gelatin mixture in the gelatin mixture solution;
lyophilizing the gelatin mixture solution including the first intermediate particle to obtain a second intermediate particle; and
cross-linking the gelatin mixture of the second intermediate particle to obtain a particle having the gelatin mixture cross-linked.

14. The method of manufacturing the anti-adhesion material according to claim 13, comprising: heating the second intermediate particle to cross-link the gelatin mixture.

## Patentansprüche

1. Antihaftmaterial, umfassend ein Partikel,
wobei das Partikel ein vernetztes Produkt einer Gelatinemischung aus einer ersten Gelatine und einer zweiten Gelatine enthält, wobei eine Kohlenwasserstoffgruppe in die erste Gelatine eingebracht wurde,
wobei das Einbringungsverhältnis der Kohlenwasserstoffgruppe in der Gelatinemischung 30 Mol-% bis 50 Mol-% beträgt,
wobei die zweite Gelatine eine Struktur aufweist, die durch die folgende Formel (1) dargestellt wird:
GltnNH-L-CHR¹R² (1)
wobei in der Formel (1) Gltn einen Gelatinerest darstellt, und L eine Einfachbindung oder eine zweiwertige Verbindungsgruppe darstellt, und R¹ eine Alkylgruppe mit 1 bis 20 Kohlenstoffatomen darstellt, und R² ein Wasserstoffatom oder eine Alkylgruppe mit 1 bis 20 Kohlenstoffatomen darstellt.

2. Antihaftmaterial nach Anspruch 1, wobei L in der Formel (1) eine Einfachbindung oder -C(O)- ist.

3. Antihaftmaterial nach Anspruch 2, wobei L in der Formel (1) eine Einfachbindung ist.

4. Antihaftmaterial nach einem der Ansprüche 1 bis 3, wobei R¹ in der Formel (1) eine lineare Alkylgruppe darstellt und R² ein Wasserstoffatom darstellt.

5. Antihaftmaterial nach einem der Ansprüche 1 bis 4, wobei das Einbringungsverhältnis der Kohlenwasserstoffgruppe der zweiten Gelatine höher ist als das der Gelatinemischung.

6. Antihaftmaterial nach einem der Ansprüche 1 bis 5, wobei das Einbringungsverhältnis der Kohlenwasserstoffgruppe der zweiten Gelatine 35 Mol-% bis 80 Mol-% beträgt.

7. Antihaftmaterial nach einem der Ansprüche 1 bis 6, wobei das Einbringungsverhältnis der Kohlenwasserstoffgruppe der Gelatinemischung 35 Mol-% bis 45 Mol-% beträgt.

8. Antihaftmaterial nach einem der Ansprüche 1 bis 7, wobei der Prozentsatz der Masse der zweiten Gelatine zur Gesamtmasse der ersten Gelatine und der zweiten Gelatine 35 Massen-% bis 90 Massen-% beträgt.

9. Antihaftmaterial nach einem der Ansprüche 1 bis 8, wobei die erste Gelatine eine alkalisch behandelte Gelatine ist.

10. Antihaftmaterial nach einem der Ansprüche 1 bis 9, wobei die erste Gelatine eine Gelatine mit einem niedrigen Endotoxingehalt ist.

11. Antihaftmaterial nach einem der Ansprüche 1 bis 10, wobei die erste Gelatine aus Kaltwasserfischen gewonnen wird.

12. Antihaftmaterial nach einem der Ansprüche 1 bis 11, das mit einem Sprühsystem auf einen betroffenen Bereich in einem lebenden Körper aufgebracht werden kann.

13. Verfahren zur Herstellung des Antihaftmaterials nach einem der Ansprüche 1 bis 12, wobei das Verfahren Folgendes umfasst:
Auflösen der Gelatinemischung aus der ersten Gelatine und der zweiten Gelatine in einem guten Lösungsmittel, um eine Gelatinemischungslösung herzustellen;
Zugeben eines schlechten Lösungsmittels zu der Gelatinemischungslösung, um ein erstes Zwischenpartikel, das die Gelatinemischung enthält, aus der Gelatinemischungslösung auszufällen;
Gefriertrocknen der Gelatinemischungslösung einschließlich des ersten Zwischenpartikels, um ein zweites Zwischenpartikel zu erhalten; und
Vernetzen der Gelatinemischung des zweiten Zwischenpartikels, um ein Partikel zu enthalten, bei dem die Gelatinemischung vernetzt ist.

14. Verfahren zur Herstellung des Antihaftmaterial nach Anspruch 13, umfassend: Erhitzen des zweiten Zwischenpartikels, um die Gelatinemischung zu vernetzen.

## Revendications

1. Matériau antiadhésif comprenant une particule,
la particule comprenant un produit réticulé d'un mélange de gélatines composé d'une première gélatine et d'une deuxième gélatine, comprenant un groupe hydrocarbure introduit dans la première gélatine ;
le rapport d'introduction du groupe hydrocarbure du mélange de gélatines allant de 30 % en moles à 50 % en moles ;
la deuxième gélatine présentant une structure représentée par la formule suivante (1) :
GltnNH-L-CHR¹R² (1)
dans laquelle formule (1), Gltn représente un résidu de gélatine, L représente une liaison simple ou un groupe de liaison divalent, R¹ représente un groupe alkyle ayant 1 à 20 atomes de carbone et R² représente un atome d'hydrogène ou un groupe alkyle ayant 1 à 20 atomes de carbone.

2. Matériau antiadhésif selon la revendication 1, dans lequel L est une liaison simple ou -C(O)- dans la formule (1).

3. Matériau antiadhésif selon la revendication 2, dans lequel L est une liaison simple dans la formule (1).

4. Matériau antiadhésif selon l'une quelconque des revendications 1 à 3, dans lequel R¹ représente un groupe alkyle linéaire, et R² représente un atome d'hydrogène dans la formule (1).

5. Matériau antiadhésif selon l'une quelconque des revendications 1 à 4, dans lequel le rapport d'introduction du groupe hydrocarbure de la deuxième gélatine est supérieur à celui du mélange de gélatines.

6. Matériau antiadhésif selon l'une quelconque des revendications 1 à 5, dans lequel le rapport d'introduction du groupe hydrocarbure de la deuxième gélatine va de 35 % en moles à 80 % en moles.

7. Matériau antiadhésif selon l'une quelconque des revendications 1 à 6, dans lequel le rapport d'introduction du groupe hydrocarbure du mélange de gélatine va de 35 % en moles à 45 % en moles.

8. Matériau antiadhésif selon l'une quelconque des revendications 1 à 7, dans lequel le pourcentage de la masse de la deuxième gélatine rapportée à la masse totale de la première gélatine et de la deuxième gélatine va de 35 % en masse à 90 % en masse.

9. Matériau antiadhésif selon l'une quelconque des revendications 1 à 8, dans lequel la première gélatine est une gélatine traitée à l'alcali.

10. Matériau antiadhésif selon l'une quelconque des revendications 1 à 9, dans lequel la première gélatine est une gélatine à faible teneur en endotoxines.

11. Matériau antiadhésif selon l'une quelconque des revendications 1 à 10, dans lequel la première gélatine est dérivée de poissons d'eau froide.

12. Matériau antiadhésif selon l'une quelconque des revendications 1 à 11, susceptible d'être appliqué sur une zone affectée d'un corps vivant à l'aide d'un système de pulvérisation.

13. Procédé de fabrication du matériau antiadhésif selon l'une quelconque des revendications 1 à 12, ledit procédé comprenant :
la dissolution du mélange de gélatines composé de la première gélatine et de la deuxième gélatine pour préparer une solution de mélange de gélatines,
l'ajout d'un solvant faible à la solution de mélange de gélatines pour précipiter une première particule intermédiaire contenant le mélange de gélatines dans la solution de mélange de gélatine,
la lyophilisation de la solution de mélange de gélatines comprenant la première particule intermédiaire pour obtenir une deuxième particule intermédiaire, et
la réticulation du mélange de gélatines de la deuxième particule intermédiaire pour obtenir une particule comprenant le mélange de gélatines réticulé.

14. Procédé de fabrication du matériau antiadhésif selon la revendication 13, comprenant : le chauffage de la deuxième particule intermédiaire pour réticuler le mélange de gélatines.
